Europäisches Patentamt

European Patent Office

Office européen des brevets

(11) Veröffentlichungsnummer: **0 126 392**
**B1**

(12) **EUROPÄISCHE PATENTSCHRIFT**

(45) Veröffentlichungstag der Patentschrift: **08.08.90**

(51) Int. Cl.⁵: **G 01 N 33/53**

(21) Anmeldenummer: **84105332.5**

(22) Anmeldetag: **11.05.84**

(54) **Träger zur Beschichtung mit immunologisch aktivem Material.**

(30) Priorität: **19.05.83 DE 3318184**

(43) Veröffentlichungstag der Anmeldung:
**28.11.84 Patentblatt 84/48**

(45) Bekanntmachung des Hinweises auf die
Patenterteilung:
**08.08.90 Patentblatt 90/32**

(84) Benannte Vertragsstaaten:
**AT BE CH DE FR GB IT LI LU NL SE**

(56) Entgegenhaltungen:
**DE-A-2 529 937**
**FR-A-2 512 453**
**US-A-4 419 453**

**PATENT ABSTRACTS OF JAPAN, Band 7, Nr.
176 (P-214)1321r, 4. August 1983; & JP-A-58 79
160**

Die Akte enthält technische Angaben, die nach
dem Eingang der Anmeldung eingereicht
wurden und die nicht in dieser Patentschrift
enthalten sind.

(73) Patentinhaber: BOEHRINGER MANNHEIM
GMBH
Sandhofer Strasse 116
D-6800 Mannheim 31 (DE)

(72) Erfinder: Batz, Hans-Georg, Dr. rer. nat.
Traubinger Strasse 63
D-8132 Tutzing (DE)
Erfinder: Hopp, Herbert
Krottenkopfstrasse 3
D-8120 Weilheim (DE)
Erfinder: Stellner, Klaus, Dr. rer. nat.
An der Freiheit 134
D-8122 Penzberg (DE)

(56) Entgegenhaltungen:
CHEMICAL ABSTRACTS, Band 72, Nr. 8, 23.
Februar 1970, Seite 28, Zusammenfassung Nr.
32541e, Columbus, Ohio, US; E. ZAHN: "Luran
S-ASA polymers: light resistant, high impact
composites"
CLINICAL CHEMISTRY, Band 26, Nr. 6, 1980,
Seiten 741-744; L.J. KRICKA et al.: "Variability in
the adsorption properties of microtitre plates
used as solid supports in enzyme
immunoassay"

Anmerkung: Innerhalb von neun Monaten nach der Bekanntmachung des Hinweises auf die Erteilung des europäischen
Patents im Europäischen Patentblatt kann jedermann beim Europäischen Patentamt gegen das erteilte europäische
Patent Einspruch einlegen. Der Einspruch ist schriftlich einzureichen und zu begründen. Er gilt erst als eingelegt,
wenn die Einspruchsgebühr entrichtet worden ist (Art. 99(1) Europäisches Patentübereinkommen).

# EP 0 126 392 B1

## Beschreibung

In der Immunologie werden häufig Kunststoffartikel benutzt, an deren Oberfläche immunologisches Material gebunden ist. Durch die Verwendung derartig beschichteter Kunststoffartikel wird die bei einem immunologischen Testverfahren üblicherweise erforderliche Trennung des gebundenen von dem nichtgebundenen Anteil erleichtert. So werden beispielsweise Radioimmunoassays und Enzymimmunoassays zur Bestimmung eines Antigens oder Haptens durchgeführt, indem man die Lösung mit dem zu bestimmenden Antigen oder Hapten in ein Kunststoffgefäß gibt, dessen Innenseite mit dem gegen das Antigen bzw. Hapten gerichteten Antikörper beschichtet ist. In das Gefäß wird ferner eine bekannte Menge des zu bestimmenden Antigens bzw. Haptens gegeben, das in charakteristischer Weise markiert ist, beispielsweise mit einem Enzym oder einem radioaktiven Material. Das zu bestimmende Antigen/Hapten und das markierte Antigen/Hapten konkurrieren un die Antikörperbindungsstellen. Es stellt sich ein Gleichgewicht zwischen dem an den Antikörper gebundenen und dem in der Lösung verbliebenen freien Anteil des Antigens bzw. Haptens ein. Die Lösung mit dem freien Anteil wird aus dem Gefäß entfernt. Der durch den Antikörper gebundene Anteil bleibt an der Gefäßwand haften und kann dort nachgewiesen werden.

Um mehrere miteinander vergleichbare Bestimmungen durchführen zu können, ist es erforderlich, daß die Beschichtung der Kunststoffgefäße mit dem entsprechenden Antikörper, falls ein Antigen bzw. Hapten nachgewiesen werden soll, bzw. mit einem Antigen oder Hapten, falls ein Antikörper nachgewiesen werden soll, mit sehr großer Gleichmäßigkeit erfolgt. Schwankt die Beschichtungsdichte von Gefäß zu Gefäß, so wirkt sich dies auf die sehr empfindliche immunologische Reaktion aus. Die Meßergebnisse werden verfälscht.

Es sind die verschiedenartigsten Träger in Form von Platten, Kugeln, Streifen, Stäbchen oder Reagensröhrchen aus den verschiedenartigsten Kunststoffen im Handel. Werden diese handelsüblichen Träger mit immunologischem Material beschichtet, so schwankt die Beschichtungsdichte und damit das bei der immunologischen Reaktion erhaltene Meßergebnis erheblich. Es werden Variationskoeffizienten beobachtet, die im allgemeinen bei den hochempfindlichen Immunotesten nicht akzeptiert werden können. In Clin. Chem. 26 (1980) 741—744 werden beispielsweise für Microtiterplatten aus Polystyrol Variationskoeffizienten von 5,2 bis 29,5% angegeben.

Es hat nicht an Versuchen gefehlt, die Variationskoeffizienten zu verbessern. So wird beispielsweise gemäß DE—OS 25 44 366 versucht, durch Glutardialdehyd-Vernetzung die Bindung des immunologischen Materials an die Trägeroberfläche zu verstärken und zu vereinheitlichen. Auch durch Variation der Beschichtungszeiten, mit Hilfe einer Vorbeschichtung bzw. Nachbeschichtung wurde versucht, eine einheitliche Beschichtungsdichte zu erzeugen (vgl. Biochemica et Biophysica Acta 492 (1977) 399—407). Darüberhinaus wird in J. Immunol. Methods 23 (1978) 23—28 sowie 47 (1981) 121—124 berichtet, daß durch Zusatz eines Detergens (Tween 20 bzw. Tween 80) eine verstärkte Adsorption des immunologischen Materials an die Trägeroberfläche erreicht wird. Ein weiterer Versuch, die Beladungsdichte gleichmäßig zu gestalten, bestand darin, das immunologische Material kovalent an die Trägeroberfläche zu binden. Dieses Prinzip ist beispielsweise in der DE—OS 27 38 138 beschrieben und beansprucht.

All diese Bemühungen führten zwar zu einer verstärkten Bindung der Antikörper bzw. des Antigens an die Kunststoffoberfläche und führten auch zu einer Verbesserung des Variationskoeffizienten. Die bischer üblichen Handelsprodukte weisen Variationskoeffizienten von 5 bis 10% auf. Es ist somit nicht gelungen, in solchem Maße eine gleichmäßige Belandungsdichte zu erzeugen, daß die mit diesen Materialien erhaltenen Meßergebnisse in der Praxis zufriedenstellen.

Aufgabe der vorliegenden Erfindung war es, einen Kunststoffträger bereitzustellen, der gleichmäßig mit immunologisch aktivem Material beschichtet werden kann.

Gelöst wird diese Aufgabe dadurch, daß im Spritzgußverfahren hergestellte Träger bereitgestellt werden, die aus im Spritzgußverfahren geformtem Kunststoff mit einem Gehalt an Hilfs- und Zusatzstoffen von weniger als 1 Gew.-% bestehen und die zur Beschichtung mit immunologisch aktivem Material verwendet werden.

Gegenstand der Erfindung ist folglich außerdem ein mit immunologisch aktivem Material beschichteter im Spritzgußverfahren hergestellter Träger, der dadurch gekennzeichnet ist, daß er aus im Spritzgußverfahren geformtem Kunststoff mit einem Gehalt an Hilfs- und Zusatzstoffen von weniger als 1 Gew.-% besteht sowie die Verwendung eines solchen Trägers bei der Durchführung von Immunoassays.

Die bisher zur Herstellung solcher Kunststoffartikel für immunologische Zwecke verwendeten Kunststoffe enthalten üblicherweise je nach Kunststoff 1—50 Gew.-% Hilfs- und Zusatzstoffe. Als solche Hilfs- und Zusatzstoffe werden üblicherweise Stabilisatoren, Gleitmittel, Weichmacher, Trennmittel, Pigmente, Füllstoffe usw. verwendet. Luran®, ein häufig zur Herstellung von Kunststoffträgern verwendeter Kunststoff, enthält beispielsweise 1—5 Gew.-%, Polyvinylchlorid sogar bis 50 Gew.-% solcher Zusatzstoffe.

Als Kunststoffe können erfindungsgemäß prinzipiell alle Kunststoffe verwendet werden, die sich zur Herstellung von Kunststoffgegenständen im Spritzgußverfahren eignen. Zur Herstellung der erfindungsgemäßen Kunststoffträger haben sich besonders vorteilhaft erwiesen: Polystyrol, Luran® bzw. Polyvinylchlorid. Es ist lediglich darauf zu achten, daß nur solche Kunststoffchargen verwendet werden, deren Hilfs- und Zusatzstoffgehalt weniger als 1 Gew-% beträgt. Vorzugsweise werden nur Luranchargen eingesetzt mit einem Hilfs- und Zusatzstoffgehalt von weniger als 0,02 Gew.-% oder Polystyrolchargen mit einem Hilfs- und Zusatzstoffgehalt von weniger als 0,05 Gew.-%.

Die Kunststoffträger werden üblicherweise im Spritzgußverfahren hergestellt. Die Spritzgußbedingungen können in den üblichen Grenzen gewählt werden, die von einem Fachmann in Abhängigkeit von dem verwendeten Kunststoff und den Spritzgußgeräten aufgrund der Herstellerangaben erarbeitet werden können. Üblicherweise wird der Druck im Bereich von 100—250 bar und die Spritztemperatur im Bereich von 190—280°C gewählt. Es ist jedoch darauf zu achten, daß der gewählte Druck und die gewählte Temperatur während des Spritzvorgangs in engen Grenzen gehalten werden. Der Druck sollte nicht mehr als ± 2 bar und die Temperatur nicht mehr als ± 2°C schwanken. Ferner ist es, um optimale Ergebnisse zu erlangen, erforderlich, daß die Oberfläche der Spritzgrußform sehr homogen ist und keine Unebenheiten aufweist und daß die Spritzgußform gleichmäßig auf eine Temperatur gekühlt wird, die vorzugsweise bei 20 bis 40°C liegt und nicht mehr als ± 1°C schwankt.

Die erfindungsgemäß hergestellten Kunststoffträger lassen sich bekannterweise mit immunologisch aktivem Material, d.h. mit Antikörpern, Antigenen und Haptenen beschichten. Hierzu wird eine Lösung des immunologisch aktiven Materials eine zur Beladung ausreichende Zeit auf die Kunststoffträger einwirken lassen. Als Lösungsmittel wird im allgemeinen Wasser benutzt, das einen geeigneten Puffer enthält.

Der pH-Wert der Lösung ist unkritisch und hängt im wesentlichen von dem Beschichtungsmaterial ab. Er liegt üblicherweise im Bereich von 5,0—8,0, vorzugsweise im neutralen Bereich. Zur vollständigen Beladung des Kunststoffträgers genügt im allgemeinen eine Einwirkungszeit von 12—24 Stunden. Die Temperatur kann im Bereich von 15—30°C gewählt werden. Sie sollte jedoch während der Inkubation nur innerhalb ± 0,5°C schwanken.

Es hat sich als zweckmäßig erwiesen, der eigentlichen Beladung eine Nachbeladung anzuschließen, bei der die mit dem immunologischen Material beschichteten Kunststoffträger einer Lösung ausgesetzt werden, die ein geeignetes Salz, beispielsweise Natriumchlorid, und ein Stabilisierungsmittel, beispielsweise Rinderserumalbumin, enthält. Zur Nachbeladung genügen 10—120 Minuten, vorzugsweise 15—60 Minuten.

Die so beschichteten Röhrchen werden steril verpackt und als solche in den Handel gebracht. Sie sind mehrere Monate stabil und können in üblichen Immunotestverfahren verwendet werden. Sie weisen einen Variationskoeffizient von <5 auf. Die mit den erfindungsgemäßen Kunststoffträgern erhaltenen Testergebnisse weisen eine deutlich geringere Schwankungsbreite auf als die Resultate, die in analoger Weise mit bisher üblichen Kunststoffträgern gewonnen worden sind.

Die Erfindung wird durch das folgende Beispiel näher erläutert:

## Beispiel

### Antikörperlösung

952 g Natriumdihydrogenphosphat-Monohydrat p.a. werden in 5 l Wasser gelöst und mit 5N Natronlauge auf pH 7,1 eingestellt. Diese Lösung wird in 165 l Wasser eingerührt. Der pH-Wert wird auf 7,1—7,4 gegebenenfalls durch Zugabe von 5N Natronlauge eingestellt.

In 500 ml dieser Pufferlösung werden 8,5 ml Antiserum- und 8,5 ml Rinderserumalbumin-Lösung (1,5 g auf 25 ml Wasser) gegeben und 30 Minuten gerührt. Die so erhaltene Antiserum-Lösung wird mit Puffer auf 2 l aufgefüllt und wiederum 30 Minuten gerührt. Diese Lösung wird mit der restlichen Pufferlösung vereinigt und erneut 30 Minuten gerührt.

### Beladung

100.000 Röhrchen aus Polystyrol 168N K21 (enthält <0,9% Zusatzstoffe) werden jeweils mit 1,5 ml ± 5% gefüllt. Die Antikörperlösung wird 16—18 Stunden bei 20 ± 0,5°C auf die Röhrchen einwirken lassen und danach abgesaugt.

### Nachbeladung

Zunächst werden 171 g Natriumchlorid p.a. 570 g Serumalbumin vom Rind Typ II in 180 l Wasser gelöst, 30 Minuten gerührt und über Nacht bei Raumtemperatur stehen gelassen. Die mit Antikörper beladenen Röhrchen werden jeweils mit 1,7 ml ± 5% der oben beschriebenen Nachbeladungslösung gefüllt. Die Lösung wird 15—30 Minuten auf die Röhrchen einwirken lassen. Danach werden die Röhrchen entleert und zweimal ausgespült und in einer Trockenbox 14—16 Stunden Getrocknet.

### Ermittlung des Variationskoeffizienten

Aus jeder Untereinheit (≙10.000 Röhrchen) einer Beladungscharge werden Willkürlich 100 Röhrchen (≙1%) gezogen und registriert. Die Röhrchen werden in zwei Gruppen zu je 50 Röhrchen für eine erste und zweite Testserie aufgeteilt. Mit den Röhrchen der ersten Testserie wird ein üblicher Enzymimmuntest durch geführt. Nach erfolgter Substratreaktion werden die Lösungen an einem Photometer gegen Substratlösung in einer Durchflußküvette vermessen und die erhaltenen Extinktionswerte registriert.

**EP 0 126 392 B1**

Aus den Einzelextinktionen $E_1$ bis $E_{50}$ wird die gemittelte Extinktion $\bar{E}$ errechnet. Die Standardabweichung S wird nach Gleichung (1)

$$(1) \quad S = \sqrt{\frac{(E_1-\bar{E})^2 + (E_2-\bar{E})^2 + \quad + (E_{50}-\bar{E})^2}{49}}$$

und der Variationskoeffizient VK nach Gleichung (2)

$$(2) \quad VK = S / \bar{E} \quad \lfloor \% \rfloor$$

ermittelt.

Für die oben beschriebenen, aus Polystyrol 168N K21 hergestellten Röhrchen ergibt sich auf diese Weise ein Variationskoeffizient von VK = 2,8%.

**Patentansprüche**

1. Im Spritzgußverfahren hergestellter Träger zur Beschichtung mit immunologisch aktivem Material, dadurch gekennzeichnet, daß er aus im Spritzgußverfahren geformtem Kunststoff mit einem Gehalt an Hilfs- und Zusatzstoffen von weniger als 1 Gew.-% besteht.

2. Träger gemäß Anspruch 1, dadurch gekennzeichnet, daß als Kunststoffe Polystyrol, Styrol-Acrylnitril-Copolymerisat, Polypropylen und/oder Polyvinylchlorid verwendet werden.

3. Träger gemäß Anspruch 1 oder 2, dadurch gekennzeichnet, daß als Kunststoff Styrol-Acrylnitril-Copolymerisat mit einem Gehalt an Hilfs- und Zusatzstoffen von weniger als 0,02 Gew.-% oder Polystyrol mit einem Gehalt an Hilfs- und Zusatzstoffen von weniger als 0,05 Gew.-% verwendet wird.

4. Verwendung von im Spritzgußverfahren hergestelltem Träger gemäß Ansprüchen 1 bis 3 zur Beschichtung mit immunologisch aktivem Material.

5. Mit immunologisch aktivem Material beschichteter im Spritzgußverfahren hergestellter Träger, dadurch gekennzeichnet, daß er aus im Spritzgußverfahren geformtem Kunststoff mit einem Gehalt an Hilfs- und Zusatzstoffen von weniger als 1 Gew.-% besteht.

6. Träger gemäß Anspruch 5, dadurch gekennzeichnet, daß als Kunststoffe Polystyrol, Styrol-Acrylnitril-Copolymerisat, Polypropylen und/oder Polyvinylchlorid verwendet werden.

7. Träger gemäß Anspruch 5 oder 6, dadurch gekennzeichnet, daß als Kunststoffe Styrol-Acrylnitril-Copolymerisat mit einem Gehalt an Hilfs- und Zusatzstoffen von weniger als 0,02 Gew.-% oder aus Polystyrol mit einem Gehalt an Hilfs- und Zusatzstoffen von weniger als 0,05 Gew.-% verwendet wird.

8. Verwendung eines Trägers gemäß einem der Ansprüche 5 bis 7 bei der Durchführung von Immunoassays.

**Revendications**

1. Support destiné à être recouvert d'une substance ayant une activité immunologique, préparé par un procédé de moulage par injection, caractérisé en ce qu'il est constitue d'une matière plastique moulée par injection, ayant une teneur en agents auxiliaires et additifs qui est inférieure à 1% en poids.

2. Support selon la revendication 1, caractérisé en ce que comme matiére plastique, on utilise du polystyrène, un copolymére styrène-acrylonitrile, du polypropylène et/ou du chlorure de polyvinyle.

3. Support selon l'une des revendications 1 ou 2, caractérisé en ce que comme matière plastique, on utilise un copolymère styrène-acrylonitrile ayant une teneur en agents auxiliaires et additifs qui est inférieure à 0,02% en poids, ou un polystyrène ayant une teneur en agents auxiliaires et additifs qui est inférieure à 0,05% en poids.

4. Utilisation d'un support préparé par un procédé de moulage par injection selon les revendications 1 à 3, pour être recouvert d'une substance ayant une activité immunologique.

5. Support préparé par un procédé de moulage par injection, recouvert d'une substance ayant une activité immunologique, caractérisé en ce qu'il est constitue d'une matière plastique moulée par injection, ayant une teneur en agents auxiliaires et additifs qui est inférieure à 1% en poids.

6. Support selon la revendication 5, caractérisé en ce que comme matière plastique, on utilise du polystyrène, un copolymère styrène-acrylonitrile, un polypropylène et/ou un chlorure de polyvinyle.

7. Support selon la revendication 5 ou 6, caractérisé en ce que comme matière plastique, on utilise un copolymère styrène-acrylonitrile ayant une teneur en agents auxiliaires et additifs qui est inférieure à 0,02% en poids, ou un polystyrène ayant une teneur en agents auxiliaires et additifs qui est inférieure à 0,05% en poids.

8. Utilisation d'un support selon l'une quelconque des revendications 5 à 7, pour la réalisation de dosages immunologiques.

4

**Claims**

1. Carrier produced in the injection moulding process for the coating with immunologically-active material, characterised in that it consists of synthetic material formed in the injection moulding process with a content of adjuvant and additive materials of less than 1 wt.%.

2. Carrier according to claim 1, characterised in that, as synthetic material, there are used polystyrene, styrene-acrylonitrile copolymer, polypropylene and/or polyvinyl chloride.

3. Carrier according to claim 1 or 2, characterised in that, as synthetic material, styrene-acrylonitrile copolymer with a content of adjuvant and additive materials of less than 0.02 wt.% or polystyrene with a content of adjuvant and additive materials of less than 0.05 wt.% is used.

4. Use of carrier according to claims 1 to 3 produced in the injection moulding process for the coating with immunologically-active material.

5. Carrier produced in the injection moulding process coated with immunologically-active material, characterised in that it consists of synthetic material formed in the injection moulding process with a content of adjuvant and additive materials of less than 1 wt.%

6. Carrier according to claim 5, characterised in that, as synthetic materials, polystyrene, styrene-acrylonitrile copolymer, polypropylene and/or polyvinyl chloride are used.

7. Carrier according to claim 5 or 6, characterised in that, as synthetic material, styrene-acrylonitrile copolymer with a content of adjuvant and additive materials of less than 0.02 wt.% or polystyrene with a content of adjuvant and additive materials of less than 0.05 wt.% is used.

8. Use of a carrier according to one of claims 5 to 7 in the carrying out of immunoassays.